(19) Europäisches Patentamt
European Patent Office
Office européen des brevets

(11) EP 1 493 442 B1

(12) **EUROPEAN PATENT SPECIFICATION**

(45) Date of publication and mention of the grant of the patent:
**28.09.2005 Bulletin 2005/39**

(51) Int Cl.[7]: **A61K 31/5375**, A61P 25/02

(21) Application number: **04023888.3**

(22) Date of filing: **22.06.2000**

(54) **Reboxetine for treating peripheral neuropathy**

Reboxetin zur Behandlung von peripheren Neuropathien

Réboxétine pour traiter les neuropathies périphériques

(84) Designated Contracting States:
**AT BE CH CY DE DK ES FI FR GB GR IE IT LI LU MC NL PT SE**
Designated Extension States:
**AL LT LV MK RO SI**

(30) Priority:
**01.07.1999 US 141968 P**
**16.07.1999 US 144131 P**
**06.10.1999 US 158256 P**
**13.12.1999 US 170381 P**

(43) Date of publication of application:
**05.01.2005 Bulletin 2005/01**

(62) Document number(s) of the earlier application(s) in accordance with Art. 76 EPC:
**00941659.5 / 1 196 172**

(73) Proprietor: **Pharmacia & Upjohn Company LLC**
**Kalamazoo, MI 49001 (US)**

(72) Inventors:
- **Ahmed, Saeeduddin**
  **Portage, Michigan 49002 (US)**
- **Birgerson, Lars**
  **Martinsville, New Jersey 08836 (US)**
- **Cetera, Pasquale**
  **Annandale, New Jersey 08801 (US)**
- **Marshall, Robert Clyde**
  **Mattawan, Michigan 49071 (US)**
- **McArthur, Robert**
  **Kalamazoo, Michigan 49009 (US)**
- **Taylor, Duncan P.**
  **Kalamazoo, Michigan 49009 (US)**
- **Wong, Erik H.F.**
  **Portage, Michigan 49024 (US)**

(74) Representative: **Duncan, Garreth Andrew et al**
**Pfizer Limited,**
**European Pharma Patent Department,**
**Ramsgate Road**
**Sandwich,**
**Kent CT13 9NJ (GB)**

(56) References cited:
**WO-A-00/00120**
**WO-A-01/47503**
**WO-A-01/62236**
**GB-A- 2 014 981**

- **MONTGOMERY S A: "The Place of Reboxetine in Antidepressant Therapy" JOURNAL OF CLINICAL PSYCHIATRY, XX, XX, vol. 59, no. SUPPL 14, 1998, pages 26-29, XP002113304 ISSN: 0160-6689**
- **ANONYMOUS: "REBOXETINE - ANOTHER NEW ANTIDEPRESSANT" DRUG AND THERAPEUTICS BULLETIN, CONSUMERS ASSOCIATION, LONDON, GB, vol. 36, no. 11, November 1998 (1998-11), pages 86-88, XP001011361 ISSN: 0012-6543**
- **BURROWS G D ET AL: "ANTIDEPRESSANT EFFICACY AND TOLERABILITY OF THE SELECTIVE NOREPINEPHRINE REUPTAKE INHIBITOR REBOXETINE: A REVIEW" JOURNAL OF CLINICAL PSYCHIATRY, XX, XX, vol. 59, no. SUPPL 14, 1998, pages 4-7, XP000989995 ISSN: 0160-6689**
- **HEALY D ET AL: "THE CLINICAL PHARMACOLOGIC PROFILE OF REBOXTINE: DOES IT INVOLVE THE PUTATIVE NEUROBIOLOGICAL SUBSTRATES OF WELLBEING ?" JOURNAL OF AFFECTIVE DISORDERS, ELSEVIER BIOCHEMICAL PRESS, AMSTERDAM, NL, vol. 51, no. 3, December 1998 (1998-12), pages 313-322, XP001012063 ISSN: 0165-0327**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 493 442 B1

## Description

## BACKGROUND OF THE INVENTION

### Field of the Invention

The present invention relates to the use of racemic reboxetine or a pharmaceutically acceptable salt thereof, the preparation of a non-transdermal medicament for the treatment or prophylaxis of peripheral neuropathy.

### Brief Description of Related Technology

Many types of depression, mental, behavioral, and neurological disorders originate from disturbances in brain circuits that convey signals using certain monoamine neurotransmitters. Monoamine neurotransmitters include, for example, norepinephrine (noradrenaline), serotonin (5-HT), and dopamine. Lower-than-normal levels of norepinephrine are associated with a variety of symptoms including lack of energy, motivation, and interest in life. Thus, a normal level of norepinephrine is essential to maintaining drive and capacity for reward.

These neurotransmitters travel from the terminal of a neuron across a small gap (*i.e.,* the synaptic cleft) and bind to receptor molecules on the surface of a second neuron. This binding elicits intracellular changes that initiate or activate a response or change in the postsynaptic neuron. Inactivation occurs primarily by transport (i.e., reuptake) of the neurotransmitter back into the presynaptic neuron. Abnormality in noradrenergic transmission results in various types of depression, mental, behavioral, and neurological disorders attributed to a variety of symptoms including a lack of energy, motivation, and interest in life. *See generally*, R.J. Baldessarini, "Drugs and the Treatment of Psychiatric Disorders: Depression and Mania" in *Goodman and Gilman's The Pharmacological Basis of Therapeutics,* McGraw-Hill, NY, NY, pp. 432-439 (1996).

Reboxetine (*i.e.*, 2-[(2-ethoxyphenoxy)(phenyl)methyl] morpholine) raises the concentration of physiologically active norepinephrine by preventing reuptake of norepinephrine, for example. Reboxetine is a norepinephrine reuptake inhibitor and has been shown to be effective in the short-term (*i.e.*, less than eight weeks) and long-term treatment of depression. In fact, reboxetine has been shown to have effectiveness that is similar to fluoxetine, imipramine, and desipramine, commonly prescribed antidepressants, in both adults and elderly patients. *See* S.A. Montgomery, *Reboxetine: Additional Benefits to the Depressed Patient,* Psychopharmocol (Oxf) 11:4 Suppl., S9-15 (Abstract) (1997).

Antidepressant drugs are sometimes divided into "generations." The first generation included the monoamine oxidase inhibitors (such as isocarboxazid and phenylhydrazine) and tricyclic agents (such as imipramine). The second generation of antidepressant drugs included compounds such as mianserin and trazodone. The third generation has included drugs called selective reuptake inhibitors (e.g., fluoxetine, sertraline, paroxetine, and reboxetine). Those drugs were characterized by relatively selective action on only one of the three main monoamine systems thought to be involved in depression (i.e., 5-HT (serotonin), noradrenaline (norepinephrine), and dopamine). APP Textbook of Psychopharmacology (A.F. Schatzberg and C.B. Nemeroff), American Psychiatric Press, 2d. ed., (1998); Lexicon of Psychiatry, Neurology and the Neurosciences (F.J. Ayd, Jr.) Williams and Wilkins (1995). The antidepressant efficacy of reboxetine is evidenced by its ability to prevent resperine-induced blepharospasm and hypothermia in mice, down regulation of β-adrenergic receptors and desensitization of noradrenaline-coupled adenylate cyclase. See M. Brunello and G. Racagni, "Rationale for the Development of Noradrenaline Reuptake Inhibitors," Human Psychophramacology, vol. 13, S-13-519, Supp. 13-519 (1998).

According to a survey by Brian E. Leonard, desipramine, maprotiline, and lofepramine are relatively selective norepinephrine reuptake inhibitors with proven efficacy. These materials increase brain noradrenaline and thereby function to relieve depression. Mianserin and mirtazepine also show antidepressant-like effects by increasing noradrenaline availability by means of blocking the pre-synaptic $\alpha_2$-adrenoceptors. Still further, oxaprotiline, fezolamine, and tomoxetine are potent and selective norepinephrine reuptake inhibitors that lack neurotransmitter receptor interactions and, thus, do not cause many of the side effects characteristic of classical tricyclic antidepressants. *See* Brian E. Leonard, "The Role of Noradrenaline in Depression: A Review," Journal of Psychopharmacology, vol. 11, no. 4 (Suppl.), pp. S39-S47 (1997)

Reboxetine also is a selective norepinephrine reuptake inhibitor, which also produces fewer of the side effects associated with the administration of classical tricyclic antidepressants. The antidepressant efficacy of reboxetine is evidenced by its ability to prevent resperine-induced blepharospasm and hypothermia in mice, down regulation of β-adrenergic receptors and desensitization of noradrenaline-coupled adenylate cyclase. *See* M. Brunello and G. Racagni, "Rationale for the Development of Noradrenaline Reuptake Inhibitors," Human Psychopharmacology, vol. 13 (Supp.) 13-519 (1998).

Reboxetine generally is described in Melloni *et al*. U.S. Patent Nos. 4,229,449, 5,068,433, and 5,391,735, and in GB-A-2,167,407. Chemically, reboxetine has two chiral centers and, therefore, exists as two enantiomeric pairs

of diastereomers, shown below as isomers (I) through (IV):

(I)

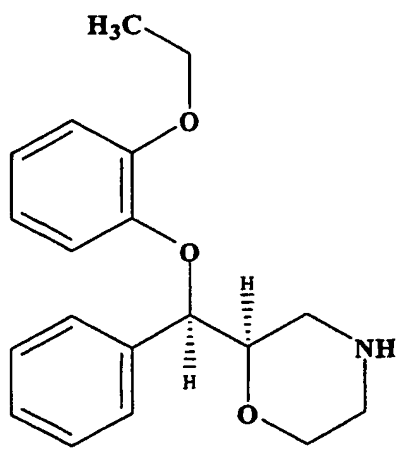

(R, R) 2-[(2-ethoxyphenoxy)(phenyl)methyl]morpholine

(II)

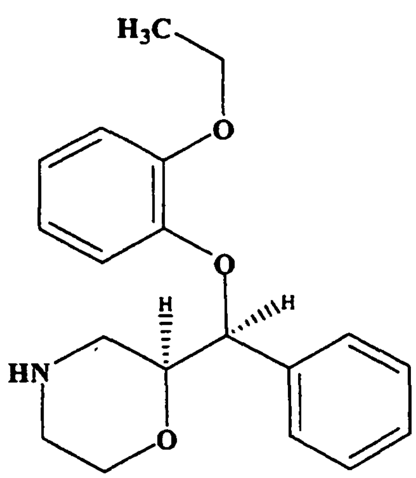

(S, S) 2-[(2-ethoxyphenoxy)(phenyl)methyl]morpholine

(III)

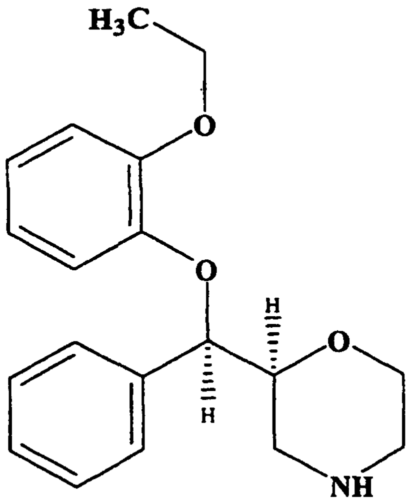

(R, S) 2-[(2-ethoxyphenoxy)(phenyl)methyl]morpholine

(IV)

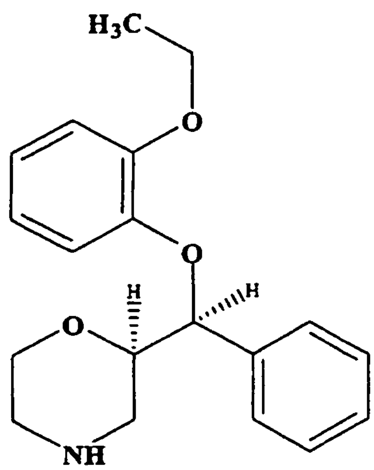

(S, R) 2-[(2-ethoxyphenoxy)(phenyl)methyl]morpholine

Many organic compounds exist in optically active forms, *i.e.*, they have the ability to rotate the plane of plane-polarized light. In describing an optically active compound the prefixes R and S are used to denote the absolute configuration of the molecule about its chiral center(s). The prefixes D and L, or (+) or (-), designate the sign of rotation of plane-polarized light by the compound, with L or (-) meaning that the compound is levorotatory. In contrast, a compound prefixed with D or (+) is dextrorotatory. There is no correlation between nomenclature for the absolute stereochemistry and for the rotation of an enantiomer. Thus, D-lactic acid is the same as (-)-lactic acid, and L-lactic acid is the same as (+)-lactic acid. For a given chemical structure, each of a pair of enantiomers are identical except that they are non-superimposable mirror images of one another. A specific stereoisomer may also be referred to as an enantiomer, and a mixture of such isomers is often called an enantiomeric, or racemic, mixture.

Stereochemical purity is important in the pharmaceutical field, where many of the most often prescribed drugs exhibit chirality. For example, the L-enantiomer of the beta-adrenergic blocking agent, propranolol, is known to be 100 times more potent than its D-enantiomer. Additionally, optical purity is important in the pharmaceutical drug field because certain isomers have been found to impart a deleterious effect, rather than an advantageous or inert effect. For example, it is believed that the D-enantiomer of thalidomide is a safe and effective sedative when prescribed for the control of morning sickness during pregnancy, whereas its corresponding L-enantiomer is believed to be a potent teratogen.

When two chiral centers exist in one molecule, there are four possible stereoisomers: (R,R), (S,S), (R,S), and (S,R). Of these, (R,R) and (S,S) are an example of a pair of enantiomers (mirror images of each other), which typically share chemical properties and melting points just like any other enantiomeric pair. The mirror images of (R,R) and (S, S) are not, however, superimposable on (R,S) and (S,R). This relationship is called diastereoisomeric, and the (S,S) molecule is a diastereoisomer of the (R,S) molecule, whereas the (R,R) molecule is a diastereoisomer of the (S,R) molecule.

Currently, reboxetine is commercially available only as a racemic mixture of enantiomers, (R,R) and (S,S) in a 1:1 ratio, and reference herein to the generic name "reboxetine" refers to this enantiomeric, or racemic, mixture. Reboxetine is commercially sold under the trade names of EDRONAX™, PROLIFT™, VESTRA™, and NOREBOX™. As previously noted, reboxetine has been shown to be useful in the treatment of human depression. Orally administered reboxetine is readily absorbed and requires once or twice a day administration. A preferred adult daily dose is in the range of about 8 to about 10 milligrams (mg). The effective daily dosage of reboxetine for a child is smaller, typically in a range of about 4 to about 5 mg. The optimum daily dosage for each patient, however, must be determined by a treating physician taking into account the patient's size, other medications which the patient may be taking, identity and severity of the particular disorder, and all of the other circumstances of the patient.

It has been reported that other antidepressants have a high pharmacological selectivity for inhibiting reuptake of norepinephrine. For example, oxaprotiline has a pharmacological selectivity with respect to inhibiting norepinephrine reuptake compared to serotonin reuptake of about 4166, based on a ratio of $K_i$ values. The corresponding pharmacological selectivity for desipramine is about 377, and that for maprotiline is about 446. *See* Elliott Richelson and Michael Pfenning, "Blockade by Antidepressants and Related Compounds of Biogenic Amine Uptake in Rat Brain Synaptosomes: Most Antidepressants Selectively Block Norepinephrine Uptake," European Journal of Pharmacology, vol. 14, pp. 277-286 (1984). Despite the relatively high selectivity of oxaprotiline, desipramine, and maprotiline, these and other known materials undesirably block receptor of other neurotransmitters to a sufficient degree that they also contribute to adverse side effects.

WO 00/00120, which was published after the priority dates of the present application, but before the filing date, discloses a transdermal formulation suitable for the treatment of pain in a subject. The transdermal formulation includes an amine-containing compound, such as racemic reboxetine

There is a need for medicaments for the treatment or prophylaxis of peripheral neuropathy containing racemic reboxetine, and the use of racemic reboxetine in the manufacture of such medicaments.

## SUMMARY OF THE INVENTION

The present invention is directed to the use of racemic reboxetine or a pharmaceutically acceptable salt, thereof, in the manufacture of a non-transdermal medicament for the treatment or prophylaxis of peripheral neuropathy.

Additional benefits and features of the present invention will become apparent to those skilled in the art from a review of the following detailed description, taken in conjunction with the example and the appended claims.

## DETAILED DESCRIPTION OF THE PREFERRED EMBODIMENTS

Reboxetine is a known compound that is active on the central nervous system, and has been used as an antidepressant. Heretofore, use of reboxetine has been limited to the treatment of depression, oppositional defiant disorder, attention-deficit/hyperactivity disorder, and conduct disorder. These proposed treatments are disclosed in International Publication Nos. WO 99/15163, WO 99/15176, and WO 99/15177. These treatment methods are limited to administration of a racemic mixture of the (S,S) and (R,R) reboxetine stereoisomers.

Reboxetine does not act like most antidepressants. Unlike tricyclic antidepressants, and even selective serotonin reuptake inhibitors (SSRIs), reboxetine is ineffective in the 8-OH-DPAT hypothermia test, indicating that reboxetine is not a SSRI. Brian E. Leonard, "Noradrenaline in basic models of depression." *European-Neuropsychopharmacol,* 7 Suppl. 1 pp. S11-6 and S71-3 (April 1997). Reboxetine is a selective norepinephrine reuptake inhibitor, with only marginal serotonin and no dopamine reuptake inhibitory activity. Reboxetine displays no anticholinergic binding activity in different animal models, and is substantially devoid of monoamine oxidase (MAO) inhibitory activity. Racemic reboxetine exhibits a pharmacological selectivity of serotonin ($K_i$)/norepinephrine ($K_i$) of about 80. The $K_i$ values are

discussed in more detail hereafter.

As used herein, the term "reboxetine" refers to the racemic mixture of the (R,R) and (S,S) enantiomers of reboxetine. In contrast, the term "(S,S) reboxetine" refers to only the (S,S) stereoisomer. Similarly, the term "(R,R) reboxetine" refers to only the (R,R) stereoisomer.

The phrases "pharmaceutically acceptable salts" or "a pharmaceutically acceptable salt thereof" refer to salts prepared from pharmaceutically acceptable acids or bases, including organic and inorganic acids and bases. Because the active compound (*i.e.*, racemic reboxetine) used in the present invention is basic, salts may be prepared from pharmaceutically acceptable acids. Suitable pharmaceutically acceptable acids include acetic, benzenesulfonic (besylate), benzoic, p-bromophenylsulfonic, camphorsulfonic, carbonic, citric, ethanesulfonic, fumaric, gluconic, glutamic, hydrobromic, hydrochloric, hydroiodic, isethionic, lactic, maleic, malic, mandelic, methanesulfonic (mesylate), mucic, nitric, oxalic, pamoic, pantothenic, phosphoric, succinic, sulfuric, tartaric, p-toluenesulfonic, and the like. Examples of such pharmaceutically acceptable salts of racemic reboxetine, thus, include, acetate, benzoate, β-hydroxybutyrate, bisulfate, bisulfite, bromide, butyne-1,4-dioate, carpoate, chloride, chlorobenzoate, citrate, dihydrogenphosphate, dinitrobenzoate, fumarate, glycollate, heptanoate, hexyne-1,6-dioate, hydroxybenzoate, iodide, lactate, maleate, malonate, mandelate, metaphosphate, methanesulfonate, methoxybenzoate, methylbenzoate, monohydrogenphosphate, naphthalene-1-sulfonate, naphthalene-2-sulfonate, oxalate, phenylbutyrate, phenylproionate, phosphate, phthalate, phylacetate, propanesulfonate, propiolate, propionate, pyrophosphate, pyrosulfate, sebacate, suberate, succinate, sulfate, sulfite, sulfonate, tartrate, xylenesulfonate, and the like. A preferred pharmaceutical salt of racemic reboxetine is methanesulfonate (*i.e.*, mesylate), which is prepared using methanesulfonic acid.

As used herein, the terms "treat," "treatment," and "treating," refer to: (a) preventing a disease, disorder, or condition from occurring in a human which may be predisposed to the disease, disorder and/or condition but has not yet been diagnosed as having it; (b) inhibiting the disease, disorder, or condition, *i.e.*, arresting its development; and (c) relieving the disease, disorder, or condition, *i.e.*, causing regression of the disease, disorder and/or condition. In other words, the terms "treat," "treatment," and "treating," extend to prophylaxis, in other words "prevent," "prevention," and "preventing," as well as treatment of established conditions. Accordingly, use of the terms "prevent," "prevention," and "preventing," would be an administration of the pharmaceutical composition to a person who has in the past suffered from the aforementioned conditions, but is not suffering from the conditions at the moment of the composition's administration. For the sake of simplicity, the term "conditions" as used hereinafter encompasses conditions, diseases, and disorders.

According to the present invention, racemic reboxetine is useful in treating peripheral neuropathy wherein inhibiting reuptake of norepinephrine provides a benefit. Racemic reboxetine may be administered, and preferably orally administered, in a sufficient amount to provide a total dose of 0.1 to 10 mg/day of the compound to an individual.

The synthesis of a racemic mixture of reboxetine is disclosed in Melloni *et al*. U.S. Patent No. 4,229,449.

While it is possible to administer racemic reboxetine or a pharmaceutically acceptable salt thereof directly without any formulation, a composition preferably is administered in the form of pharmaceutical medicaments comprising racemic reboxetine or a pharmaceutically acceptable salt thereof. The composition can be administered in oral unit dosage forms such as tablets, capsules, pills, powders, or granules. The inventive composition also can be introduced parenterally, (e.g., subcutaneously, intravenously, or intramuscularly), using forms known in the pharmaceutical art. The inventive composition further can be administered rectally or vaginally in such forms as suppositories or bougies.

It may be desirable or necessary to introduce the composition or pharmaceutical compositions containing the selective norepinephrine reuptake inhibitor to the brain, either directly or indirectly. Direct techniques usually involve placement of a suitable drug delivery catheter into the ventricular system to bypass the blood-brain barrier. One such suitable delivery system used for the transport of biological factors to specific anatomical regions of the body is described in U.S. Patent No. 5,011,472.

In general, the preferred route of administering the composition is oral, with a once or twice a day administration. The dosage regimen and amount for treating patients with the composition is selected in accordance with a variety of factors including, for example, the type, age, weight, sex, and medical condition of the patient, the severity of the condition, the route of administration and the particular compound employed, reboxetine racemate. An ordinarily skilled physician or psychiatrist can readily determine and prescribe an effective (*i.e.*, therapeutic) amount of the compound to prevent or arrest the progress of the condition. In so proceeding, the physician or psychiatrist could employ relatively low dosages at first, subsequently increasing the dose until a maximum response is obtained.

Pharmaceutical compositions suitable for oral administration can be of any convenient form, such as sachets, tablets, capsules, pills, or aerosol sprays, each containing a predetermined amount of the active compound either as a powder or granules, or as a solution or a suspension in an aqueous liquid, a non-aqueous liquid, an oil-in-water emulsion, or a water-in-oil liquid emulsion. Such compositions can be prepared by any method that includes the step of bringing the active compound either into intimate association with a carrier, which constitutes one or more necessary or desirable ingredients. Generally, the compositions are prepared by uniformly and intimately admixing the active

ingredient with liquid carriers or finely divided solid carriers or both, and then, if necessary, shaping the product into a desired form.

For example, a tablet can be prepared by compression or molding techniques, optionally, using one or more accessory ingredients. Compressed tablets can be prepared by compressing the active ingredient in a suitable machine into a free-flowing form, such as a powder or granules. Thereafter, the compressed, free-flowing form optionally can be mixed with a binders, diluents, lubricants, disintegrating agents, effervescing agents, dyestuffs, sweeteners, wetting agents, and non-toxic and pharmacologically inactive substances typically present in pharmaceutical compositions. Molded tablets can be made by molding a mixture of the powdered compound moistened with an inert liquid diluent in a suitable machine.

Suitable binders for use in the pharmaceutical preparation include, for example, starches, gelatine, methylcellulose, gum arabic, tragacanth, and polyvinylpyrrolidone. Suitable diluents for use in the pharmaceutical preparation include, for example, lactose, dextrose, sucrose, mannitol, sorbitol, and cellulose. Suitable lubricants for use in the pharmaceutical preparation include, for example, silica, talc, stearic acid, magnesium or calcium stearate, and or polyethylene glycols. Suitable disintegrating agents for use in the pharmaceutical preparation include, for example, starches, alginic acid, and alginates. Suitable wetting agents for use in the pharmaceutical preparation include, for example, lecithin, polysorbates, and laurylsulfates. Generally, any effervescing agents, dyestuffs, and/or sweeteners known by those of ordinary skill in the art can be used in the preparation of a pharmaceutical composition.

According to the present invention racemic reboxetine is effective in the treatment of child, adolescent, and adult patients. For purposes of the present invention, a child is considered to be a person below the age of puberty, an adolescent is considered to be a person between the age of puberty and up to about 18 years of age, and an adult generally is a person of at least about 18 years of age. As previously noted, the optimum daily dosage for each patient must be determined by a treating physician taking into account each patient's size, other medications which the patient is taking, identity and severity of the disorder, and all of the other circumstances of the patient.

As stated above, reboxetine acts as an antidepressant. Reboxetine, however, does not act like most antidepressants. Unlike trycyclic antidepressants, and even selective serotonin reuptake inhibitors (SSRIs), reboxetine is ineffective in the 8-OH-DPAT hypothermia test, indicating that reboxetine is not a selective serotonin reuptake inhibitor. Rather, reboxetine is selective for the noradrenergic system. Reboxetine is not an SSRI, but is a novel, selective, noradrenalinc reuptake inhibitor (NRI). B. Leonard, "Noradrenaline in basic models of depression." *European-Neuropsychopharmacol,* 7 Suppl. 1 pp. S11-6 and S71-3 (Apr., 1997). Unlike most prior generation drugs, reboxetine is a highly selective norepinephrine reuptake inhibitor, with only marginal serotonin and no dopamine reuptake inhibitory activity. Reboxetine displays no anticholinergic binding activity in different animal models, and is devoid of monoamine oxidase (MAO) inhibitory activity.

Reboxetine also is a highly potent, pharmacologically specific, and fast acting agent. Investigations indicate that reboxetine has potent antireserpine activity, and combines the inhibitory properties of classical tricyclic antidepressants on the reuptake of noradrenaline with an ability to desensitize β-adrenergic receptor function, without showing any appreciable blocking action at muscarinic, cholinergic, histaminergic, and α-adrenergic receptors. Moreover, reboxetine shows less vagolytic activity than tricyclic antidepressants, and no evidence of cardiotoxicity.

According, to the present invention, racemic reboxetine can be used to treat or prevent peripheral neuropathy. Specifically, reboxetine has been found particularly useful for treating or enhancing the treatment or prevention of peripheral neuropathy, with greater efficacy and with fewer side effects than with treatment by known drugs.

The Mental and neurological disorder that may be treated or prevented by administration of a therapeutically effective amount of a racemic reboxetine (or a pharmaceutically acceptable salt thereof) according to the present invention is peripheral neuropathy.

The racemate form of reboxetine is well tolerated and has a wide safety range. Racemic reboxetine can be administered to an individual in an amount in a range of 2 to 20 milligrams per patient per day (mg/day), and preferably 4 to 10 mg/day, and more preferably 6 to 10 mg/day. Depending upon the formulation and the individual's disorder, the total daily dosage can be administered in small amounts up to two times a day. Reboxetine typically is administered orally, for example, in the form of tablets, but can be adminstered parentally, rectally, or vaginally.

A preferred method of administering racemic reboxetine is oral dosing once or twice a day. It can also be administered at dosages of 2, 4, 6, 8, 10, or 12 mg/day or fractions thereof. For example, suitable administrations could be 4 mg in the morning and 2 or 4 mg in the afternoon or evening. In some patients, the ideal dosing would be 3 to 5 mg in the morning and 3 to 5 mg in the afternoon. A skilled physician or psychiatrist can determine the precise level of dosing. The ideal dosing is routinely determined by an evaluation of clinical trials and the needs of specific patients.

In accordance with the present invention, the racemic reboxetine also can be administered as the free base or a pharmaceutically acceptable salt thereof. The phrases "pharmaceutically acceptable salts" or "a pharmaceutically acceptable salt thereof" refer to salts prepared from pharmaceutically acceptable acids or bases, including organic and inorganic acids and bases as described above. A preferred pharmaceutical salt of reboxetine is methanesulfonate (*i.e.*, mesylate), which is prepared using methanesulfonic acid.

Treatment or prevention of above disorder involves the administration of reboxetine in a manner and form that result in a reduction in the symptoms of the disease or disorder. Typically, the symptoms exhibited by children, adolescents, and adults are similar to each other. Hence, as noted above, methods of the present invention are effective in the treatment of child, adolescent, and adult patients.

## EXAMPLE

This example demonstrates the superior pharmacological selectivity and potency of a composition according to the present invention.

Sprague-Dawley rats weighing about 250 to about 300 grams (g) were decapitated, and cerebral cortical tissue was removed immediately. Cerebral cortices were homogenized in nine volumes of medium each containing 0.32 molar (M) sucrose using a rotating pestle. The obtained homogenate was centrifuged at about 1000 x g for about 10 minutes at about 4°C. A supernatant was collected and further centrifuged at about 20,000 x *g* for about 20 minutes at a temperature of about 4°C. A protein pellet resulting from the centrifuge steps was re-suspended in a Kreb's-Hepes buffer to result in a protein concentration of about 2 mg/ml of buffer. The buffer was maintained at a pH of about 7.0 and contained: 20 mM Hepes; 4.16 mM $NaHCO_3$; 0.44 mM $KH_2PO_4$; 0.63 mM $NaH_2PO_4$; 127 mM NaCl; 5.36 mM KCl; 1.26 mM $CaCl_2$; and 0.98 mM $MgCl_2$.

Protein/buffer suspension was introduced into 166 assay tubes such that about 30 μg ($10^{-6}$ grams) to about 150 μg of the protein was added to each of 166 assay tubes (*i.e.*, 80 assays per transporter assay). Binding to serotonin and norepinephrine reuptake sites was determined as follows. Synaptosomal uptake of $^3$H-norpinephrine was determined as follows. About 1.4 nanomolar of [$^3$H]citalopram and about 1.9 nM of [$^3$H]nisoxetine were used to label serotonin and norepinephrine reuptake sites, respectively. Nonspecific binding was defined by 100 micromolar (μM) fluoxetine (for serotonin) and 10 μM desipramine (for norepinephrine). Incubation in total assay volume of about 500 microliters (μl) was carried out for about 60 minutes (for serotonin) and 120 minutes (for norepinephrine). Both incubations were carried out at about 25°C, and terminated by rapid filtration through a 48-well cell harvester though GFB filters (pre-soaked with about 0.5 PEI for about 4 hours) in a 3 x 5 ml of ice-cold 200 mM tris-HCl, pH 7.0. Punched-out filters were placed into 7 ml minivials and radioactive assayed by liquid scintillation counting.

The ability of reboxetine (*i.e.*, racemic mixture of (R,R) and (S,S) reboxetine), (R,R) reboxetine, and (S,S) reboxetine to bind to norepinephrine and serotonin reuptake sites was evaluated in binding assays using the two radioligands, [$^3$H]citalopram and [$^3$H]nisoxetine. The concentration of the test compound required to inhibit 50% of the specific binding at the two reuptake sites ($IC_{50}$ values) were determined by non-linear least square regression analysis. A conversion of $IC_{50}$ values to $K_i$ values was performed using the Cheng-Prassoff equation presented below:

$$K_i = IC_{50}/(1 + ([L]/[K_d \text{ of } L])),$$

wherein [L] is the radioligand concentration used in nM, and $K_d$ is the binding affinity of L in nM. *See* Y.C. Cheng and W.H. Prusoff, "Relationship Between the Inhibitory Constant ($K_i$) and the Concentration of Inhibitor Which Causes 50% Inhibition ($IC_{50}$) of an Enzymatic Reaction," Biochemical Pharmacology, vol. 22, pp. 3099-3108 (1973).

The $K_i$ values calculated according to the Cheng-Prassoff equation are provided in the table below:

Table

| **Compound** | **Norepinephrine Reuptake ($K_i$ nM)** | **Serotonin Reuptake ($K_i$ nM)** | **Selectivity of $K_i$ of Serotonin/ Norepinphrine** |
|---|---|---|---|
| (S,S) Reboxetine | 0.23 ± 0.06 | 2937 ± 246 | 12,770 |
| (R,R) Reboxetine | 7.0 ± 1.7 | 104 ± 43 | 15 |
| Reboxetine | 1.6 ± 0.6 | 129 ± 13 | 81 |

The data shows that racemic reboxetine has an 81 fold selectivity favoring norepinephrine reuptake inhibition over serontonin reuptake inhibition.

## Claims

1. The use of racemic reboxetine, or a pharmaceutically acceptable salt thereof, in the manufacture of a non-transdermal medicament for the treatment or prophylaxis of peripheral neuropathy.

2. The use of claim 1, wherein the racemic reboxetine is to be administered in an amount of 2 to 20 mg/day.

3. The use of claim 2, wherein the racemic reboxetine is to be administered in an amount of 4 to 10 mg/day.

4. The use of claim 3, wherein the racemic reboxetine is to be administered in an amount of 6 to 10 mg/day.

5. The use of any preceding claim, wherein the pharmaceutically acceptable salt of racemic reboxetine is the methanesulfonate.

## Patentansprüche

1. Verwendung von racemischem Reboxetin oder eines seiner pharmazeutisch akzeptablen Salze zur Herstellung eines nicht-transdermalen Arzneimittels zur Behandlung oder Prävention von peripheren Neuropathien.

2. Verwendung nach Anspruch 1, wobei das racemische Reboxetin in einer Menge von 2 - 20 mg/Tag verabreicht wird.

3. Verwendung nach Anspruch 2, wobei das racemische Reboxetin in einer Menge von 4 - 10 mg/Tag verabreicht wird.

4. Verwendung nach Anspruch 3, wobei das racemische Reboxetin in einer Menge von 6 - 10 mg/Tag verabreicht wird.

5. Verwendung nach einem der vorhergehenden Ansprüche, wobei das pharmazeutisch annehmbare Salz des racemischen Reboxetins Methansulfonat ist.

## Revendications

1. Utilisation de réboxétine racémique, ou d'un de ses sels pharmaceutiquement acceptables, dans la production d'un médicament non transdermique pour le traitement ou la prophylaxie de la neuropathie périphérique.

2. Utilisation suivant la revendication 1, dans laquelle la réboxétine racémique est destinée à être administrée en une quantité de 2 à 20 mg/jour.

3. Utilisation suivant la revendication 2, dans laquelle la réboxétine racémique est destinée à être administrée en une quantité de 4 à 10 mg/jour.

4. Utilisation suivant la revendication 3, dans laquelle la réboxétine racémique est destinée à être administrée en une quantité de 6 à 10 mg/jour.

5. Utilisation suivant l'une quelconque des revendications précédentes, dans laquelle le sel pharmaceutiquement acceptable de réboxétine racémique est le méthanesulfonate.